Europäisches Patentamt

European Patent Office

Office européen des brevets

(19)

(11) Publication number: **0 224 331 B1**

# EUROPEAN PATENT SPECIFICATION

(12)

(45) Date of publication of patent specification: **23.10.91**

(51) Int. Cl.⁵: **C12N 15/00**, C07H 21/04, C12N 1/20, C12P 21/00, //(A01N63/00,C12N15:00, C12R1:07),(C12N1/20, C12R1:19)

(21) Application number: **86308180.8**

(22) Date of filing: **21.10.86**

(54) **Production of insecticidal protein of bacillus thuringiensis subsp. aizawai IPL by the expression of insecticidal protein gene in host cells.**

(30) Priority: **28.10.85 JP 242528/85**
**06.02.86 JP 24563/86**
**25.02.86 JP 40925/86**

(43) Date of publication of application:
**03.06.87 Bulletin 87/23**

(45) Publication of the grant of the patent:
**23.10.91 Bulletin 91/43**

(84) Designated Contracting States:
**BE CH DE FR GB IT LI NL SE**

(56) References cited:
**EP-A- 0 063 949**
**EP-A- 0 093 062**
**EP-A- 0 153 166**
**EP-A- 0 186 379**
**WO-A-86/01536**

(73) Proprietor: **SUMITOMO CHEMICAL COMPANY, LIMITED**
**Kitahama 4-chome 5-33**
**Chuo-ku Osaka 541(JP)**

(72) Inventor: **Oeda, Kenji**
**5-1, Honmachi 9-chome**
**Toyonaka Osaka 560(JP)**
Inventor: **Oshie, Kazuyuki**
**8-6, Kasayacho**
**Nishinomiya Hyogo 663(JP)**
Inventor: **Shimizu, Masatoshi**
**15-10-106, Kusunokicho**
**Ashiya Hyogo 659(JP)**
Inventor: **Nakamura, Keiko**
**868-215 Kotsukayama Tamon-cho**
**Tarumi-ku Kobe Hyogo 650(JP)**
Inventor: **Ohkawa, Hideo**
**1-8 Sakasedai C-604**
**Takarazuka Hyogo 665(JP)**

(74) Representative: **Myerscough, Philip Boyd et al**
**J.A.Kemp & Co. 14, South Square Gray's Inn**
**London, WC1R 5EU(GB)**

## Description

The present invention relates to the production of an insecticidal protein of Bacillus thuringiensis subsp. aizawai IPL by the DNA recombinant technology. particularly, it relates to a gene coding for the insecticidal protein of Bacillus thuringiensis subsp. aizawai IPL, which has a potent insecticidal activity against noxious insects belonging to the order, Lepidoptera, such as larvae of diamond-back moth (Plutella maculipennis) and cotton cutworm (Spodoptera litura), and to an expression plasmid carrying the said gene and being capable of expressing the insecticidal protein in host cells. It also relates to a transformant microorganism harboring the said expression plasmid and being capable of producing the insecticidal protein, and to a process for producing the insecticidal protein comprising culturing the said transformant microorganism in a suitable medium.

It has been known that microorganisms classified as Bacillus thuringiensis produce l-2 μm in length of crystals of insecticidal protein in their sporulation phase, and that the insects that have eaten the protein stop eating and die from rupture of their intestines. Microorganisms belonging to Bacillus thuringiensis are further classified into 29 subspecies according to the flagella antigen, the esterase pattern and the like, and each of them exhibits a distinct and particular insecticidal activity.

The present inventors have studied aiming at the utilization, as insecticidal agents, of the insecticidal protein of Bacillus thuringiensis subsp. aizawai IPL, which exhibits a potent insecticidal activity against noxious insects belonging to the order, Lepidoptera, in particular, larvae of diamond-back moth and cotton cutworm which are harmful to vegetable crops, and, as a result, have completed the present invention.

Specifically, the inventors have succeeded in cloning of a gene coding for the insecticidal protein of Bacillus thuringiensis subsp. aizawai IPL having a strong insecticidal activity against larvae of diamond-back moth and cotton cutworm, and elucidated the entire DNA sequence of 3465 base pairs (bp) of the structural gene coding for the insecticidal protein, as well as the primary structure of the insecticidal protein. The inventors have also constructed expression plasmids by inserting the said gene into various expression vectors and created transformant microorganisms by transforming microorganisms with each of the said expression plasmids.

In the accompanying drawings,

Fig. I is a diagram of the construction of the expression plasmids pAH7, pAH8 and pTBI. Black boxes indicate the gene coding for the insecticidal protein, boxes with vertical lines lac promoter, boxes with horizontal lines ribosome RNA terminator, white boxes tac promoter and dotted boxes lacZ gene. ATG and TAA indicate the initiation codon and stop codon of the insecticidal protein gene, respectively. Ah, Kp, Pv, Bm, Hc and Ps indicate restriction enzymes Aha III, Kpn I, Pvu II, Bam HI, Hinc II and Pst I, respectively.

Fig. 2 shows the entire DNA sequence of the insecticidal protein gene consisting of 3465 base pairs. The upper lines indicate the DNA sequence and the lower the deduced amino acid sequence. The region from the base No. I through No. 3465 is the structural gene coding for the insecticidal protein.

Fig. 3 is a restriction map of the insert DNA [22.4 Kilo base(kb)] of the plasmid pAB6. The insecticidal protein gene of B. thuringiensis subsp. aizawai IPL is indicated by open boxes. A detailed restriction map of the gene is given in the lower box.

Fig. 4. shows the results of the quantitative analysis of the insecticidal protein produced by the transformant E. coli with a densitometer. (I), (2) and (3) are the results of the measurements of the insecticidal proteins in the crude extract of transformant E. coli JMI03/pAH7, JMI03/pAH8 and JMI03/pTBI, respectively. The arrows indicate the peaks of the bands of the insecticidal protein.

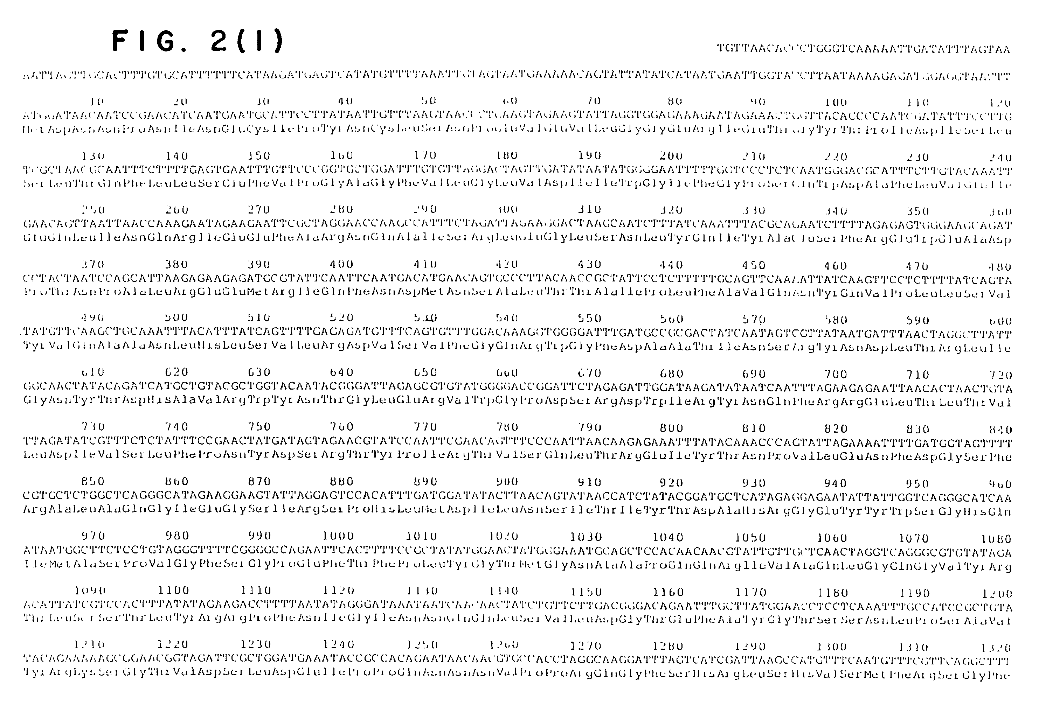

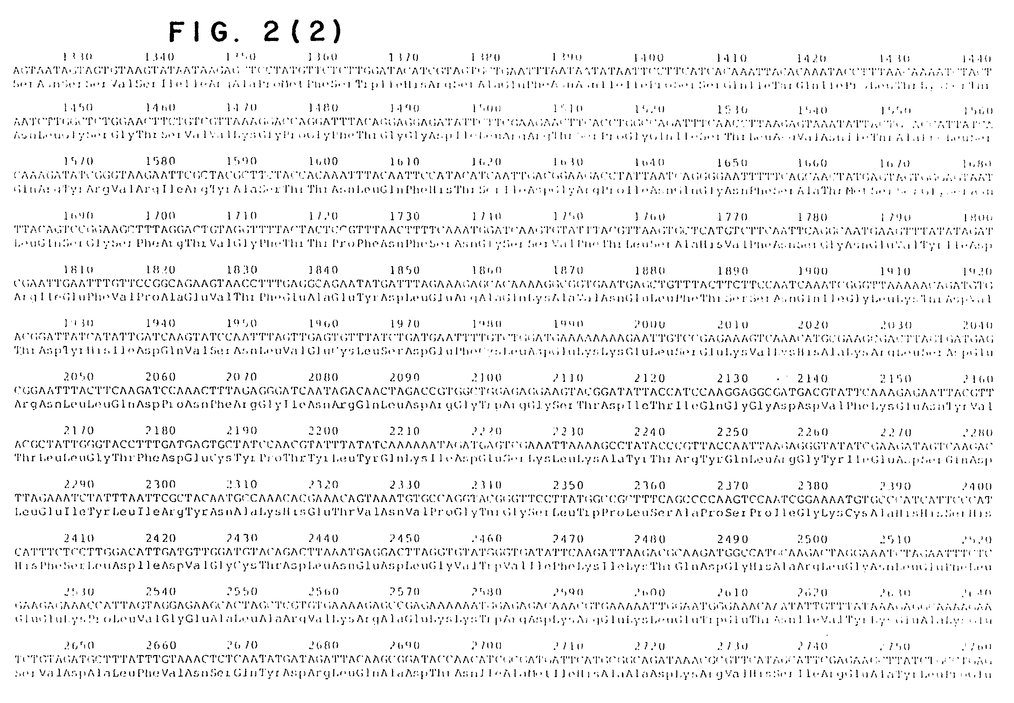

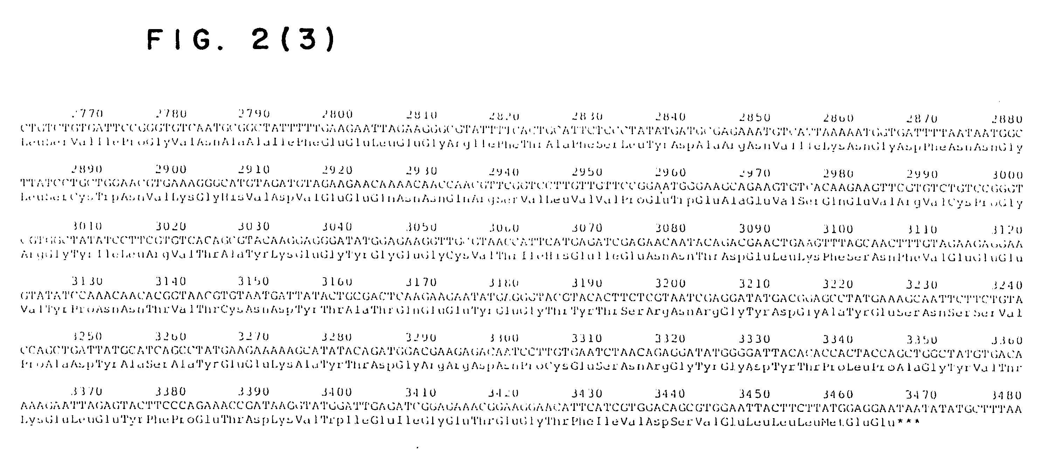

Further, the inventors have provided a method for producing the insecticidal protein of Bacillus thuringiensis subsp. aizawai IPL in a large scale, which method is characterized by culturing such a transformant microorganism. The insecticidal protein gene of Bacillus thuringiensis subsp. aizawai IPL can be identified by the DNA sequence given in Fig. 2, or its deduced amino acid sequence given in Fig. 2.

A plasmid containing the insecticidal protein gene of the present invention can be obtained by first preparing a gene library from the plasmid DNA of Bacillus thuringiensis subsp. aizawai IPL and then screening the library with a suitable probe, for example, a synthetic oligonucleotide designed based on the DNA sequence given in Fig. 2, or the DNA sequence of an insecticidal protein gene of Bacillus thuringiensis HD-I-Dipel, which is known to closely relates to Bacillus thuringiensis subsp. aizawai IPL, for the isolation of the plasmid containing the insecticidal protein gene of the present invention.

In preparing the recombinant plasmid carrying the insecticidal protein gene of the present invention, it is preferable to select a clone of N. thuringiensis subsp. aizawai IPL exhibiting a strong insecticidal activity as a source of the gene.

As is well know, with respect to various amino acids, the DNA sequence coding for a particular amino acid is not limited to a single codon, but there are plural DNA sequence for one amino acid. Likewise, the DNA sequence coding for the amino acid sequence of the insecticidal protein of B. thuringiensis subsp. aizawai IPL is not limited only to the naturally occuring one but there are many possible DNA sequences coding for the insecticidal protein. It should be understood that the present invention includes not only the natural DNA sequence coding for the insecticidal protein of B. thuringiensis subsp. aizawai IPL elucidated by the present invention as given in Fig. 2, but also other DNA sequences which code for the amino acid sequences of the insecticidal protein of B. thuringiensis subsp. aizawai IPL elucidated by the present inventors.

Moreover, it is known that it is possible to artificially introduce some changes in the DNA sequence in a given gene without altering the substantial properties to be expressed by the gene, or rather with an improvement of such properties. In the case of the insecticidal protein gene of the present invention, which, as mentioned above, may be of the naturally occuring sequence or artificial ones, it is possible to artificially introduce some additions, deletions or substitutions in the DNA sequence to create equivalent or improved insecticidal protein genes. It should be understood that the present invention emcompasses the naturally occuring sequence, as well as such modified sequences.

The expression vector plasmids which enable microorganisms, such as E. coli, to produce the insecticidal protein of B. thuringiensis subsp. aizawai IPL in the cells can be constructed by inserting the gene coding for the insecticidal protein of B. thuringiensis subsp. aizawai IPL into a suitable expression vector such as pUCl8 containing lac promoter (Pharmacia), pKK223-4 containing tac promoter of E. coli, as well as the terminator of rrbB ribosome RNA, pDR720 having trp promoter (Pharmacia), and an inducible expression vector pPL-Lambda (Pharmacia).

By the transformation of host cells such as E. coli, e.g., E. coli JMl03 strain (Pharmacia), with each of the expression vector plasmids obtained as above, the transformant cells which produce the insecticidal protein in the cells are prepared. The thus prepared transformant cells are grown in a suitable medium under suitable conditions to produce the insecticidal protein in large scale. This process can advantageously be performed by adding an inducer, isopropylthiogalactoside in the culture medium at an early stage of the cultivation.

After the cultivation, the produced insecticidal protein can easily be recovered as condensed aggregates by, for example, ultrasonification of the cells, followed by centrifugation.

For the production of the insecticidal protein, not only the E. coli-vector system, but also any of other microorganism-vector systems, such as Bacillus subtilis, Saccharomyces cerevisiae, Pseudomonas and Actinomycetes-vector systems may also be used taking the advantage of each of the host-vector systems.

The following examples are given to illustrate the invention more precisely. These examples are not intended to limit the present invention in any way. It is to be understood that the invention includes any modifications or improvements thereof.

Isolation of the insecticidal protein gene

Selection of B. thuringiensis subsp. aizawai IPL No. 7 having a potent insecticidal activity against insects belonging to the order, Lepidoptera, such as larvae of diamond-back moth and cotton cutworm.

Step I: Purification of B. thuringiensis subsp. aizawai IPL

B. thuringiensis subsp. aizawai IPL (maintained at the U. S. Department of Agriculture Research Service) was grown on a PY plate culture medium [tryptone (Difco) l0 g/l, NaCl (Nakarai Chemical) 5 g/l, yeast extract (Difco) 5 g/l, agar (Difco) l2 g/l] to give 32 clones in pure culture. The plasmid analysis of the clones was conducted as follows: Each clone was grown in l0 ml of a PY liquid medium (the same composition as that of the PY plate culture medium except that no agar was used) for 24 hours and the cells were harvested by centrifugation (8,000 × g, l0 minutes). A plasmid DNA was isolated from the cells according to the method of Birnboim and Doly [Nucleic Acid Res. 7, l5l3-l523 (l979)] and analyzed by 0.4 % agarose gel electrophoresis to find that no single common plasmid pattern was observed with respect to the 32 clones, and that they are divided into at least 9 groups according to their plasmid patterns.

The clone with which l2 plasmid DNA bands of 4.0 Md, 4.8 Md, 5.4 Md, 8.5 Md, l2 Md, l5 Md, l7 Md, 2l Md, 37 Md, 40 Md, 50 Md, and 52 Md were observed was named as Bacillus thuringiensis subsp. aizawai IPL No. 7, and deposited at the Fermentation Research Institute, Japan, under the deposition No. FERM P-8654, and also under the deposition NO. FERM BP-ll50 according to the BUDAPEST TREATY ON THE INTERNATIONAL RECOGNITION OF THE DEPOSIT OF MICROORGANISMS FOR THE PURPOSES OF PATENT PROCEDURE.

Step 2: Selection of B. thuringiensis subsp. aizawai IPL No. 7 by insecticidal activity test

The clone of each of the 9 groups of purified B. thuringiensis subsp. aizawai IPL strain was grown on PY culture medium plates (l5 cm in diameter) at 30 °C for 7 days. After the formation of spores and crystals of the insecticidal protein was confirmed with a phase contrast microscope, the cells were harvested and the freeze-thawing of the cells was repeated 3 times. The cells were suspended in 2 ml of distilled water and subjected to ultrasonification to give the crystals of the insecticidal protein in suspension.

A laboratory feed soaked with l ml of the suspension of the crystals, or its l0- or l00-fold dilution was given to a group of l0 cotton cutworm larvae (4th instar). After 3 days, the number of the dead insects was counted. The same test was conducted on larvae of diamond-back moth (3rd instar). When l ml of the suspension of the crystals of B. thuringiensis subsp. aizawai IPL No. 7 ($2.2 \times 10^7$ spores in l ml) was given to l0 larvae of diamond-back moth, all the l0 insects were dead. When l0 cotton cutworms were added to a suspension of the crystals ($2.6 \times 10^8$ spores in l ml), all the l0 insects were dead. Thus, the result proved that the B. thuringiensis subsp. aizawai IPL No. 7 produces a potent insecticidal activity against both of the noxious insects.

Cloning of the gene coding for the insecticidal protein of B. thuringiensis aizawai IPL No. 7

Step l: Synthesis of a DNA probe

A synthetic DNA probe (5'-CACAAATCCAGCACCGGG-3') was designed in the light of the DNA sequence of the insecticidal protein gene of Bacillus thuringiensis subsp. kurstaki HD-l Dipel [Whiteley et al., J. Biol. Chem. 260,6264-72 (l985)]. After the DNA oligomer was synthesized with a DNA synthesizer Model 380A (Applied Biosystems), l ml of 27 % ammonium hydroxide was added to the DNA in a collecting vial and heated at 55 °C for 4 hours. The mixture was dried with a condensor. The dried DNA was dissolved in l00 $\mu$l of 0.0lM triethylamine-acetic acid (TEAA)(pH 7.2), which was then applied to the reversed phase HPLC column Cl8 and eluted with a mixed solvent of acetonitrile-0.lM TEAA (pH 7.2). The fractions of an absorption peak at 260 nm were collected and dried. After addition of l00 $\mu$l of 80 % acetic acid in acetonitrile, the mixture was allowed to stand for l5 minutes to turn to pale orange. The mixture was then dried up and combined with l00 $\mu$l of 0.0lM TEAA (pH 7.2) and l00 $\mu$l of ethyl acetate. The ethyl acetate layer was removed off from the mixture, and l00 $\mu$l of diethyl ether was added instead After the same procedures were repeated twice, the mixture was again dried up. The residues were dissolved in 0.0lM TEAA (pH 7.2) and applied to HPLC. The fraction of the absorption peak at 260 nm was collected and dried. The residues were again dissolved in a mixture of l0 mM Tris-HCl (pH 7.5) and l mM EDTA (TE) solution. The thus prepared DNA probe was then labelled with $^{32}$P.

After 5 $\mu$l of the DNA probe prepared as above (about l00 p mole), l5 units of E. coli polynucleotide kinase (Takara Shuzo), l00 micro-Ci of [gamma-$^{32}$P]ATP (Amersham Japan) and l0 $\mu$l of l0-fold concentration of a reaction mixture [0.5 M Tris-HCl (pH 7.6), 0.l M $MgCl_2$, and 0.l M 2 mercaptoethanol] were combined, the mixture was made up to l00 $\mu$l with distilled water, and allowed to react at 37 °C for l hour. After the reaction mixture was admixed with a mixed solvent of chloroform and phenol (l:l), the supernatant was applied to a DE-52 column (Whatman)(0.5 ml bed size) equilibrated with TE buffer (pH 7.5). The column was washed with 3 ml of TE buffer (pH 7.5) and eluted with 0.7M NaCl-TE buffer (pH 7.5). The radioactive fractions were collected to give the $^{32}$P labelled DNA probe.

Step 2: Preparation of the plasmid DNA library of B. thuringiensis subsp. aizawai IPL No. 7

B. thuringiensis subsp. aizawai IPL No. 7 was grown in 200 ml of a PY liquid medium, and the harvested cells were washed with 30 ml of G buffer [l0% glycerol, l mM EDTA and 50 mM Tris-HCl (pH 8.0)] and suspended in 8 ml of lysozyme (5 mg/ml). The mixture was allowed to react at 30 °C for 2 hours. After l6 ml of an alkaline solution [0.2 N NaOH and l % sodium dodecyl sulfate (SDS)] was added thereto, the mixture was mixed gently and allowed to stand at room temperature for l0 minutes. Then, l2 ml of a neutralizing solution [3 M sodium acetate (pH 4.8)] was added to the mixture, and the mixture was allowed to stand at 4 °C for l.5 hours. After centrifugation (l4,000 rpm, 20 minutes), 70 ml of chilled ethanol was added to the supernatant, and the mixture was kept at -20 °C for l hour to precipitate DNA. The DNA was collected, dried up and suspended in 5 ml of 0.l M sodium acetate. The suspension was treated twice with phenol equilibrated with TE buffer (pH 7.5). After the supernatant was collected, 2 volumes of chilled ethanol was added to it to precipitate DNA. The dried DNA was resuspended in 6 ml of TE buffer (pH 7.5) and the plasmid DNA was purified by CsCl equilibrium density gradient centrifugation. The plasmid DNA (5 $\mu$g) was digested with l0 units of restriction enzyme Bam HI. The same volume of a mixed solvent of phenol and chloroform (l:l) was added to the mixture to yield a supernatant. Ethanol was added to the supernatant to precipitate DNA. The DNA was dried up and suspended in 20 $\mu$l of TE buffer (pH 7.5).

On the other hand, 2 $\mu$g of a pUC 8 vector plasmid (Pharmacia) was digested with 5 units of a restriction enzyme, Bam HI, and a DNA was recovered by the same procedures as above, and suspended in 20 $\mu$l of TE buffer (pH 7.5). To this suspension, 5 $\mu$l of E. coli alkaline phosphatase (Takara Shuzo, 2.0 units), 50 $\mu$l of 0.l M Tris-HCl buffer (pH 8.0) and l5 $\mu$l of distilled water were added, and incubated at 60°C for l hour. The DNA was recovered by treating the reaction mixture twice with phenol-chloroform and precipitating the DNA from the isolated supernatant with ethanol, and suspended in 20 $\mu$l of TE buffer (pH 7.5).

To a mixture of l5 $\mu$l of the plasmid DNA Bam HI fragments and l5 $\mu$l of the Bam HI fragments of the pUC 8 vector DNA, were added l $\mu$l of T4 DNA ligase (Takara Shuzo,0.l unit), 7.5 $\mu$l of 0.l M dithiothreitol (Nakarai Chemical), 7.5 $\mu$l of l0 mM adenosine triphosphate (Nakarai Chemical), 25 $\mu$l of a 3-fold concentration reaction mixture [200 mM Tris-HCl (pH 7.6) and 20 mM $MgCl_2$] and 5 $\mu$l of distilled water until the mixture had the total volume of 75 $\mu$l. The mixture was then incubated at l4°C for 6 hours. The resulting ligase reaction mixture (l0 $\mu$l) was added to l00 $\mu$l of a suspension of competent cells of E. coli DHl (ATCC 33849), which had been prepared by the method of Scott et al., [Cell Technology, 2, 6l6-626, (l983)]. The mixture was incubated at 0°C for l5 minutes and heated at 42°C for 40 seconds. After 0.9 ml of an L broth liquid medium [tryptone l0 g, yeast extract 5 g, NaCl 5 g, glucose (Nakarai Chemical) l g and distilled water equal to l liter] was added, the cells were incubated at 37°C for l hour and grown on an L broth plate medium (l.2 % agar in the L broth liquid medium) containing a final concentration of 50 $\mu$g/ml of ampicillin.

Step 3: Isolation of a clone carrying the insecticidal protein gene by colony hybridization

The colonies spread on a plate were replicated on 2 nitrocellulose filters, which were then placed on a plate culture medium containing ampicillin (50 $\mu$g/ml) and chloramphenicol (600 $\mu$g/ml) and incubated overnight. Each of the filter was treated with 2.5 ml/filter of 0.5 N NaOH for 5 minutes and air-dried. The same volume of l M Tris-HCl (pH 7.5) was added to the filter and allowed to stand for 5 minutes. After being air-dried, the filter was treated with 2.5 ml of l M Tris-HCl (pH 7.5)-l.5 M NaCl for 5 minutes, air-dried and treated at 80°C for 3 hours under vacuum. After addition of l0 ml/4 filters of 0.l % SDS-4 × SSC [SSC: 0.l5 M NaCl - 0.0l5 M sodium citrate (pH 7.5)], the filters were treated at 60°C for l5 minutes. The colonies on the filters were wiped off and the filters were immersed in 6 ml of prehybridization solution [4 × SSC, l0 × Denhart, l00 $\mu$g/ml salmon testis single stranded DNA (Sigma); wherein l0 × Denhart is a solution containing 0.2 % ficoll, 0.2 % polyvinylpyrrolidone and 0.2 % BSA) and treated at 60°C for 3 hours. The $^{32}$P labeled synthetic DNA probe prepared in Step l was added to the prehybridization solution, and the filters were placed in the mixture and incubated at 56°C overnight. After hybridization, the filters were washed with 4 × SSC- 0.l % SDS solution at 56°C for l5 minutes for 4 times and dried. The filters were placed on X-ray films to conduct radioautography. The positive colonies were collected from the master plate and the colony hybridization of the colonies was performed in the same manner as above to isolate a positive clone which was named as E. coli DHl/pAB6.

Analysis of the insecticidal protein gene of B. thuringiensis subsp. aizawai IPL No. 7

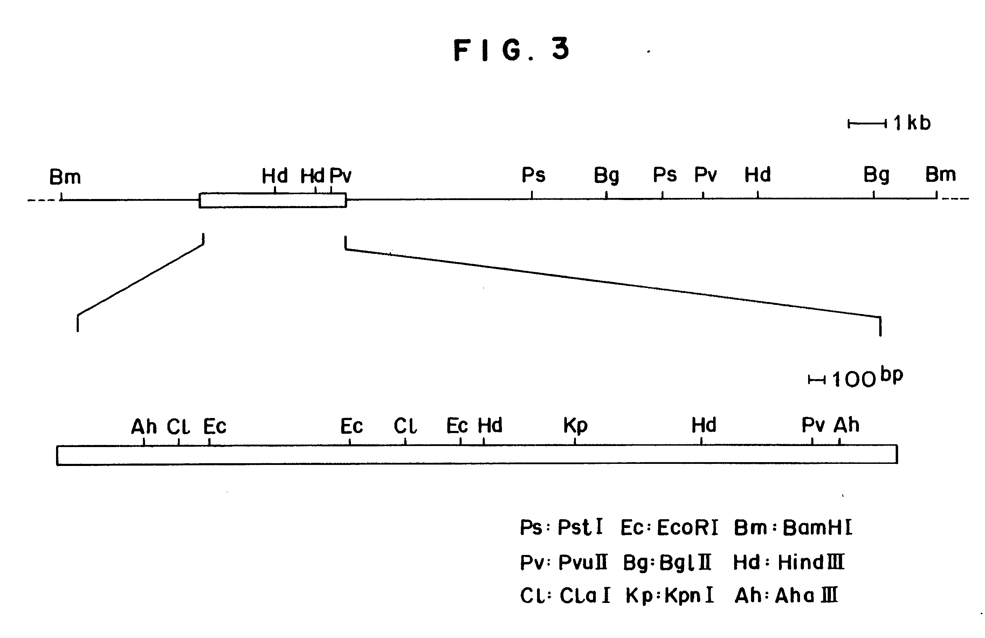

The plasmid pAB6 was isolated from the thus isolated positive clone E. coli DHl/pAB6 according to the method of Birnboim and Doly. The restriction map (Fig. 3, upper part) of the insert DNA (22.4 kb) was prepared by digesting the plasmid pAB6 with various restriction enzymes including Bam HI, Hind III, Pst I, Bgl II, Pvu II, Eco RI, Aha III, Kpn I and Cla I, and analyzed by 0.7 % agarose gel electrophoresis. By using the synthetic DNA probe prepared as above, Southern hybridization [J. Mol. Biol., 98 503-5l7 (l975)] was carried out to identify the region coding for the insecticidal protein gene in the insert DNA, and the detailed restriction map of the region was prepared (Fig. 3 lower part).

After DNA fragments obtained by digesting the DNA were sub-cloned in vector pUC8, the plasmid DNAs containing these DNA fragments were isolated by the method of Birnboim and Doly. Each of the isolated DNAs was suspended in l8 $\mu$l of TE buffer (pH 7.5) and 2 $\mu$l of 2 N NaOH was added to the suspension, which was then allowed to stand at room temperature for 5 minutes. Following the addition of 8 $\mu$l of 7.5 M ammonium acetate, l00 $\mu$l of chilled ethanol was added to the mixture to precipitate DNA. The DNA was recovered by centrifugation (l2,000 rpm, 5 minutes), dried and dissolved in distilled water in a concentration of 0.5 pmole/5 ml. After l.5 $\mu$l of l0-fold concentration of Klenow-buffer (Takara Shuzo), l $\mu$l of primer DNA (P-L Biochemicals) and 4.5 $\mu$l of distilled water was added to 5 $\mu$l of the plasmid DNA(5 pmole) prepared as above, to make the total volume l2 $\mu$l, the mixture was heated at 60°C for l5 minutes and allowed to stand at room temperature for 20 minutes. The reaction mixture was mixed with 2 $\mu$l of [alpha-$^{32}$P] ATP (400 Ci/mmole, Amersham Japan)

and I µl of Klenow fragment enzyme (Takara Shuzo, 2 units).

The mixture (3.2 µl) was added to 4 kinds of dNTP + ddNTP mixture (Takara Shuzo, 2 µl each) and allowed to stand at 42°C for 20 minutes. After I µl of a Chase solution (Takara Shuzo) was added to each of the resulting mixtures, the mixtures were allowed to stand at 42°C for 20 minutes. After addition of 6 µl of 95 % formamide staining solution (0.I % bromophenol and 0.I % xylenecyanol), the mixtures (2 µl) were applied to 6 % acrylamide-urea gel, which was prepared by a conventional method, and electrophoresis at I700 V for 6 hours. The gel was dried and placed on X-ray films. After the films were processed, the DNA sequences were determined.

The DNA sequence of the insecticidal protein gene of B. thuringiensis subsp. aizawai IPL No. 7 is given in Fig. 2. As shown in Fig. 2, the gene has 3465 bp of coding region starting from ATG and ending at the stop codon TAA and encodes II55 amino acids.

I. Construction of expression plasmids pAH8 and pAH7 for expression of the insecticidal gene of B. thuringiensis subsp. aizawai IPL No. 7 in E. coli cells

Step I: Preparation of DNA Aha III fragment

Approximately I0 µg of the recombinant plasmid pAB6 carrying the insecticidal protein gene was allowed to react with ca. 30 units of restriction enzyme Aha III at 37°C for I hour in 30 µl of Aha III reaction solution [I0 mM Tris-HCl (pH 7.5), 60 mM NaCl, 7 mM MgCl₂, I0 mM EDTA and I mM dithiothreitol]. The reaction mixture was subjected to gel electrophoresis on 0.8 % low melting point agarose gel (Bethesda Research Laboratory) containing 0.I µg/ml of ethidium bromide. The portion of the gel containing 3.5 kb of the DNA Aha III fragment was isolated under ultraviolet rays, placed in a test tube and heated at 65°C for 5 minutes. TE buffer [I0 mM Tris-HCl (pH 8.0), 0.5 mM EDTA] (2 times the volume of the used gel) was added into the tube and the mixture was extracted with phenol saturated with TE buffer. After centrifugation at I0,000 rpm for 5 minutes, the upper layer was combined with I/40 volume of 4 M NaCl and 2 volumes of ethanol, and allowed to stand at -80°C for I0 minutes to precipitate DNA. The DNA was collected by centrifugation at I0,000 rpm for I0 minutes and suspended in I0 µl of distilled water.

Step 2: Preparation of a vector

Expression vector pUC I8 (Pharmacia) I µg was digested with I unit of a restriction enzyme, Hinc II (Takara Shuzo) at 37°C for I hour in 20 µl of Hinc II reaction solution [I0 mM Tris-HCl (pH 7.4), I00 mM NaCl, 7 mM MgCl₂ and I0 mM dithiothreitol]. The same volume of a mixed solvent of phenol-chloroform (I:I) was added to the reaction mixture. After being stirred, the mixture was centrifuged at I0,000 rpm for 5 minutes to yield a supernatant, to which 2 volumes of chilled ethanol was added. The mixture was then kept at -80°C for I5 minutes and centrifugated at I0,000 rpm for I0 minutes. The precipitated DNA was collected and suspended in I0 µl of distilled water.

Step 3: Construction of expression plasmids pAH7 and pAH8

The DNA Aha III fragment obtained in Step I and the expression vector pUC I8 prepared in Step 2, I µg each, were mixed and reacted with 7.2 units of T4 DNA ligase (Takara Shuzo) at I6°C for 2 hours in 45 µl of a ligase reaction solution [66 mM Tris-HCl (pH 7.6), 6.6 mM MgCl₂, I0 mM dithiothreitol and I.0 mM ATP]. With this reaction mixture, E. coli JMI03 (Pharmacia) was transformed in accordance with the method of Cohen et al. [Proc. Natl. Acad. Sci. USA., 69, 2II0-2II4 (I972)]. The ampicillin resistant colonies were cultured and the plasmid DNA was isolated according to the method of Birnboim et al.

Approximately I µg of the plasmid DNA was digested with 3 units of restriction enzyme, Hind III, at 37°C for I hour in a Hind III reaction solution [I0 mM Tris-HCl (pH 7.5), 60 mM NaCl, I0 mM MgCl₂, I mM 2-mercaptoethanol and I00 µg/ml bovine serum albumin] and analyzed on agarose gel electrophoresis. The expression plasmid in which the insecticidal protein gene is ligased in the same direction as the lac promoter of the vector was named as pAH8. The expression plasmid in which the gene is ligased in the opposite direction was named as pAH7 (See Fig. I).

2. Construction of expression plasmid pTBI for use in expression of the insecticidal protein gene of B. thuringiensis subsp. aizawai No. 7 in E. coli cells

Step I: Preparation of Pst I - Bam HI fragment containing the insecticidal protein gene

Approximately 5 µg of the expression plasmid pAH8 containing the insecticidal protein gene was digested with about 20 units of a restriction enzyme, Pst I, and about 20 units of restriction enzyme, Bam HI, at 37°C for I hour in 50 µl of a Pst I reaction solution [I0 mM Tris-HCl (pH 7.5), 50 mM NaCl, I0 mM MgCl₂, I mM 2-mercaptoethanol and I00 µg/ml bovine serum albumin]. The reaction mixture was mixed with 30 µl of phenol saturated with TE buffer for phenol extraction. The mixture was centrifuged at 10,000 rpm for 5 minutes and the upper layer was collected. After addition of 1/40 volume of 4 M NaCl and 2 volumes of ethanol, the layer was allowed to stand at -80°C for 10 minutes and then centrifuged at 10,000 rpm for 10 minutes to recover DNA. The recovered DNA was dried and suspended in 20 μl of distilled water.

Step 2: Construction of a vector

Approximately 5 μg of expression vector pKK223-3 (Pharmacia) was digested with 0.1 unit of a restriction enzyme, Bam HI, (Takara Shuzo) at 37°C for 15 minutes in a Bam HI reaction solution [10 mM Tris-HCl (pH 8.0), 7 mM MgCl₂, 100 mM NaCl, 2 mM 2-mercaptoethanol and 0.01 % bovine serum albumin]. The reaction mixture was supplied to 0.8 % low melting point agarose gel containing 0.1 μg/ml of ethidium bromide. The portion of the gel wherein the vector DNA (4.6 kb) resulting from the cleavage at one of the two Bam HI sites of the vector pKK 223-3 was possibly contained was isolated under ultraviolet rays, placed in a test tube and heated at 65°C for 5 minutes to melt the gel. The DNA was recovered from the gel by phenol extraction and ethanol precipitation, and suspended in 20 μl of distilled water.

To this DNA suspension, a final concentration of 3 mM of 4 kinds of deoxynucleotides and 5 units of E. coli DNA polymere I large fragment were added, and the mixture was reacted at 25°C for 2 hours in 60 μl of a Hind III reaction solution [10 mM Tris-HCl (pH 7.5), 60 mM NaCl, 10 mM MgCl₂, 1 mM 2-mercaptoethanol and 100 μg/ml bovine serum albumin]. DNA was recovered by phenol extraction and ethanol precipitation, and suspended in 20 μl of distilled water. Approximately 1 μg of the DNA obtained as above was allowed to react with 3 units of T4 DNA ligase at 16°C for 2 hours in 45 μl of a ligase reaction solution [66 mM Tris-HCl (pH 7.6), 6.6 mM MgCl₂, 10 mM dithiothreitol and 1.0 mM ATP]. With the reaction mixture, E. coli JM103 cells were transformed in accordance with the method of Cohen et al. The colonies resistant to ampicillin were grown and the plasmid DNA was isolated from the transformants according to the method of Birnboim et al. Approximately 1 μg of the plasmid DNA was digested with 3 units of restriction enzyme Bam HI at 37°C for 1 hour in a Bam HI reaction solution and the reaction mixture was subjected to the agarose gel electrophoresis analysis. By analysis of the plasmid DNA, the plasmid wherein the Bam HI site in the multicloning sites of the pKK223-3 plasmid was retained, but the other Bam HI site was vanished, was selected and named as pKK223-4.

Approximately 5 μg of the pKK223-4 DNA was digested with 10 units of restriction enzyme Pst I and 10 units of restriction enzyme Bam HI at 37°C for 1 hour in 50 μl of a Pst I reaction solution [10 mM Tris-HCl(pH 7.5), 50 mM NaCl, 10 mM MgCl₂, 1 mM 2-mercaptoethanol and 100 μg/ml of bovine serum albumin]. DNA was recovered by phenol extraction and ethanol precipitation, and suspended in 20 μl of distilled water.

Step 3: Construction of expression plasmid pTBI

The Pst I - Bam HI DNA fragment obtained in Step 1 and the vector pKK223-4 obtained in Step 2, 1 μg each, were combined and the mixture was allowed to react with 7.2 units of T4 DNA ligase (Takara Shuzo) at 16°C for 2 hours in 45 μl of a ligase reaction solution [66 mM Tris-HCl (pH 7.6), 6.6 mM MgCl₂, 10 mM dithiothreitol and 10 mM ATP]. With the reaction mixture, E. coli JM103 was transformed according to the method of Cohen et al.

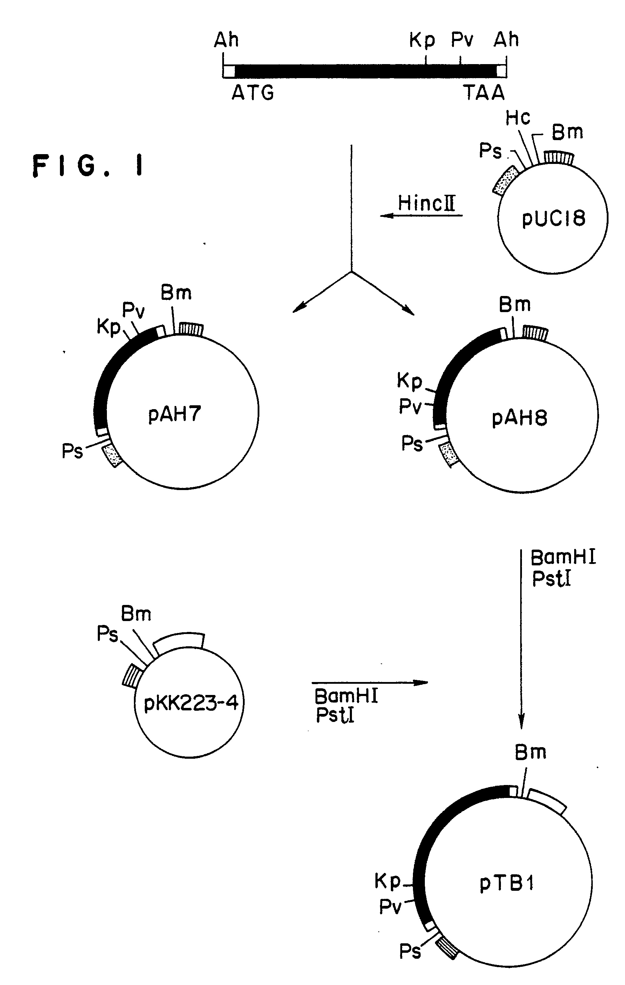

The colonies resistant to ampicillin were grown and the plasmid DNA was isolated. Approximately 1 μg of the plasmid DNA was digested with 3 units of a restriction enzyme, Bam HI, at 37°C for 1 hour in 30 μl of a Bam HI reaction solution and the reaction mixture was subjected to agarose gel electrophoresis analysis. The plasmid wherein the insecticidal protein gene was inserted downstream of the tac promoter of the expression vector was selected and named as expression plasmid pTBI (See Fig. 1).

3. Production of the insecticidal protein in E. coli

In accordance with the method of Cohen et al., E. coli JM103 cells were transformed with each of the expression plasmids pAH7, pAH8 and pTBI.

The analysis of the insecticidal protein of B. thuringiensis subsp. aizawai IPL produced by the resulting transformant E. coli JM103/pAH7, JM103/pAH8 and JM103/pTBI was carried out as follows:

Each of the transformants was grown overnight in an L broth liquid medium [tryptone (Difco) 10 g, NaCl 5 g, yeast extract (Difco) 5 g and distilled water equal to 1 liter]. The resulting culture medium, 0.1 ml, was inoculated to 10 ml of an L broth liquid medium and cultured at 37°C. When the OD 660 nm was reached to 0.1, a final concentration of 2 mM of isopropylthiogalactoside was added to the medium. After being cultivated at 37°C for 20 hours, 0.3 ml of the culture medium was centrifuged (3,000 rpm, 15 minutes) to harvest the cells. The cells were suspended in 50 μl of a sample buffer [62.5 mM Tris-HCl (pH 8.0), 2 % (w/w) sodium dodecyl sulfate, 5 % (v/v) 2-mercaptoethanol, 10 % (w/v) glycerol and 0.01 %

bromophenol blue]. After being heated at 100°C for 5 minutes, the suspension was applied to SDS-polyacrylamide gel electrophoresis according to the method of Laemmli [Nature 227, 680-685 (1970)]. The gel was stained with coomassie brilliant blue, destained, dried and fixed on a filter paper. A band of the insecticidal protein of molecular weight of 125K daltons, which was allowed to cross-react with anti-insecticidal protein antibody (IgG), was observed with the gels of the transformant E. coli JM103 cells containing the expression plasmids.

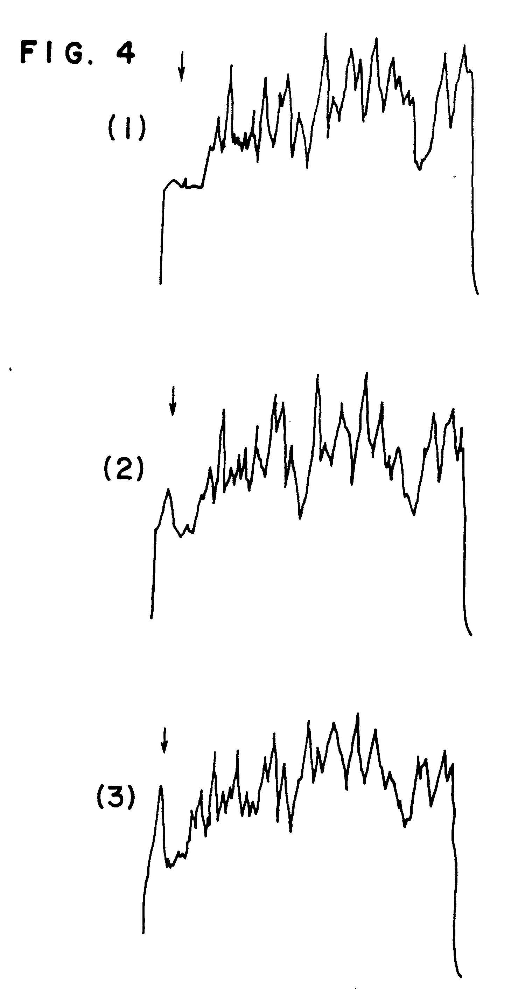

The measurement of the protein bands on the gels with a densitometer indicated that E. coli JM103/pAH7, JM103/pAH8 and JM103/pTB1 produced the insecticidal protein in amounts of 1%, 8% and 35% of the total protein, respectively (See Fig. 4). Thus, it was confirmed that these transformants of E. coli effectively produced the insecticidal protein of B. thuringiensis aizawai IPL.

### 4. Isolation of the insecticidal protein produced in the transformant E. coli cells

The transformant E. coli cells were grown overnight in an L broth liquid medium, and 0.1 ml of the culture medium was inoculated to 10 ml of an L broth medium. The culture was incubated at 37°C until the OD 660 nm value reached to 0.1, when a final concentration of 2 mM of isopropylthiogalactoside was added to the medium. After being further cultivated, 5 ml of the culture medium was centrifuged at 3,000 rpm for 15 minutes to harvest the cells. The cells were frozen at -80°C and thawed at room temperature. This freeze-thawed procedure was repeated 3 times. The cells were suspended in 2 ml of TE buffer [10 mM Tris-HCl (pH 7.5) and 1 mM EDTA] and ultrasonificated for 30 seconds for 3 times.

The resulting crude extract was centrifuged at 7,000 rpm for 10 minutes to yield precipitates. The precipitates were resuspended in the sample buffer for electrophoresis and analyzed on SDS-PAGE to find that about 80% of the total protein of the precipitates was the insecticidal protein. Thus it was confirmed that the insecticidal protein can effectively be isolated by the procedures described above. It was also confirmed that these procedures can be applied to a large volume culturing.

### Claims

1. A DNA containing the DNA sequence coding for the insecticidal protein of Bacillus thuringiensis subsp. aizawai IPL wherein the DNA sequence codes for a protein having the amino acid sequence given in Fig. 2.

2. A DNA according to Claim 1 wherein the DNA sequence is as given in Fig. 2.

3. A structural gene coding for the insecticidal protein of Bacillus thuringiensis subsp. aizawai IPL having the amino acid sequence given in Fig. 2.

4. A plasmid carrying a DNA sequence coding for the insecticidal protein of Bacillus thuringiensis subsp. aizawai IPL having the amino acid sequence given in Fig. 2.

5. A plasmid according to Claim 4 which is named as pAB6 and comprises pUC8 containing, at its Bam HI site, a 22.4 kb Bam HI fragment from a plasmid DNA obtainable from FERM-BP-1150 and carrying the insecticidal protein gene given in Figure 2.

6. A recombinant expression vector carrying a DNA sequence coding for the insecticidal protein of Bacillus thuringiensis subsp. aizawai IPL having the amino acid sequence of Fig. 2, the vector being capable of producing the insecticidal protein in host cells.

7. A recombinant expression vector according to Claim 6 wherein the DNA sequence is as given in Fig. 2.

8. A recombinant expression vector according to Claim 6 which is named as pAH8 or pAH7 and comprises pUC18 containing, at its Hinc II site, a 3.5 kb Aha III fragment obtainable from the 22.4 kb Bam HI fragment as defined in claim 5, and wherein the insecticidal protein gene is in the same direction as the lac promoter of pUC18 in the case of pAH8, or is in the opposite direction from the lac promoter of pUC18 in the case of pAH7.

9. A recombinant expression vector according to claim 6 which is named as pTB1 and comprises the Bam HI Pst I fragment carrying the insecticidal protein gene of pAH8, as defined in claim 8, that fragment being cloned into the Bam HI Pst I multiple cloning site of pKK223-3, the second Bam site of the said pKK223-3 having previously been destroyed by being cut and filled in.

10. A microorganism which belongs to E.coli carrying a recombinant plasmid or expression vector as claimed in any one of Claims 5 to 9.

11. A process for producing the insecticidal protein of Bacillus thuringiensis subsp. aizawai IPL, which comprises culturing a microorganism as claimed in Claim 10 in a suitable medium and collecting the protein from the culture.

**Revendications**

1. ADN contenant la séquence d'ADN codant pour la protéine douée de propriétés insecticides de Bacillus thuriengiensis sous-espèce aizawai IPL, la séquence d'ADN codant pour une protéine ayant la séquence d'acides aminés donnée à la figure 2.

2. ADN suivant la revendication 1, dans laquelle la séquence d'ADN est donnée à la figure 2.

3. Gène de structure codant pour la protéine douée de propriétés insecticides de Bacillus thuriengiensis sous-espèce aizawai IPL ayant la séquence d'acides aminés donnée à la figure 2.

4. Plasmide portant une séquence d'ADN codant pour la protéine douée de propriétés insecticides de Bacillus thuriengiensis sous-espèce aizawai IPL ayant la séquence d'acides aminés donnée à la figure 2.

5. Plasmide suivant la revendication 4, qui est dénommé pAB6 et qui comprend pUC8 contenant, à son site Bam HI, un fragment Bam HI de 22,4 kb provenant d'un ADN plasmidique qui peut être obtenu à partir de FERM-BP-1150 et qui porte le gène de la protéine douée de propriétés insecticides donné à la figure 2.

6. Vecteur d'expression recombinant portant une séquence d'ADN codant pour la protéine douée de propriétés insecticides de Bacillus thuriengiensis sous-espèce aizawai IPL ayant la séquence d'acides aminés de la figure 2, le vecteur étant susceptible de produire la protéine douée de propriétés insecticides dans des cellules hôtes.

7. Vecteur d'expression recombinant suivant la revendication 6, dans lequel la séquence d'ADN est telle que donnée à la figure 2.

8. Vecteur d'expression recombinant suivant la revendication 6 qui est dénommé pAH8 ou pAH7 et qui comprend pUC18 contenant, à son site Hinc II, un fragment Aha III de 3,5 kb qui peut être obtenu à partir du fragment Bam HI de 22,4 kb, tel que défini à la revendication 5, et dans lequel le gène de la protéine douée de propriétés insecticides est dans le même sens que le promoteur lac de pUC18 dans le cas de pAH8, ou dans le sens opposé du promoteur lac de pUC18 dans le cas de pAH7.

9. Vecteur d'expression recombinant suivant la revendication 6 qui est dénommé pTB1 et qui comprend le fragment allant de Bam HI à Pst I et portant le gène de la protéine douée de propriétés insecticides de pAH8, tel que défini à la revendication 8, ce fragment étant cloné dans le site de clonage multiple de pKK223-3, allant de Bam HI à Pst I, le second site Bam du pKK223-3 ayant été au préalable détruit en étant coupé et rempli.

10. Microorganisme qui appartient à E. Coli portant un plasmide recombinant ou un vecteur d'expression recombinant tel que revendiqué à l'une quelconque des revendications 5 à 9.

11. Procédé de production de la protéine douée de propriétés insecticides de Bacillus thuriengiensis sous-espèce aizawai IPL, qui consiste à cultiver un microorganisme tel que revendiqué à la revendication 10 dans un milieu convenable et à recueillir la protéine de la culture.

**Patentansprüche**

1. DNA, enthaltend die DNA-Sequenz, die das insektizide Protein von Bacillus thuringiensis subsp. aizawai IPL codiert, wobei die DNA-Sequenz ein Protein, das die in Fig. 2 dargestellte Aminosäuresequenz aufweist, codiert.

2. DNA nach Anspruch 1, wobei die DNA-Sequenz die in Fig. 2 dargestellte ist.

3. Strukturgen, das das insektizide Protein von Bacillus thuringiensis subsp. aizawai IPL codiert, das die in Fig. 2 dargestellte Aminosäuresequenz aufweist.

4. Plasmid, das eine DNA-Sequenz trägt, die das insektizide Protein von Bacillus thuringiensis subsp. aizawai IPL codiert, das die in Fig. 2 dargestellte Aminosäuresequenz aufweist.

5. Plasmid nach Anspruch 4, das als pAB6 bezeichnet wird und pUC8 umfaßt, das an seiner BamHI-Stelle ein 22,4 kb BamHI-Fragment von einer Plasmid-DNA, erhältlich aus FERM-BP-1150, enthält und das in Fig. 2 dargestellte insektizide Proteingen trägt.

6. Rekombinanter Expressionsvektor, der eine DNA-Sequenz trägt, die das insektizide Protein von Bacillus thuringiensis subsp. aizawai IPL codiert, das die Aminosäuresequenz von Fig. 2 aufweist, wobei der Vektor zur Herstellung des insektiziden Proteins in Wirtszellen befähigt ist.

7. Rekombinanter Expressionsvektor nach Anspruch 6, wobei die DNA-Sequenz die in Fig. 2 dargestellte ist.

8. Rekombinanter Expressionsvektor nach Anspruch 6, der als pAH8 oder pAH7 bezeichnet wird und pUC18 umfaßt, der an seiner HincII-Stelle ein 3,5 kb AhaIII-Fragment enthält, das aus dem in Anspruch 5 definierten 22,4 kb BamHI-Fragment erhältlich ist, und in dem, im Fall von pAH8, das insektizide Proteingen in der gleichen Richtung wie der lac-Promotor von pUC18 oder, im Fall von pAH7, in der entgegengesetzten Richtung vom lac-Promotor von pUC18 liegt.

9. Rekombinanter Expressionsvektor nach Anspruch 6, der als pTB1 bezeichnet wird und das BamHI/PstI-Fragment umfaßt, das das in Anspruch 8 definierte, insektizide Proteingen von pAH8 trägt, wobei das Fragment in die multiple BamHI/PstI-Clonierungsstelle von pKK223-3 cloniert wird und die zweite BamHI-Stelle von pKK223-3 zuvor durch Spaltung und Auffüllung zerstört worden ist.

10. Mikroorganismus, der zu E.coli gehört und ein rekombinantes Plasmid oder einen Expressionsvektor nach einem der Ansprüche 5 bis 9 trägt.

11. Verfahren zur Herstellung des insektiziden Proteins von Bacillus thuringiensis subsp. aizawai IPL, das die Züchtung eines Mikroorganismus nach Anspruch 10 in einem geeigneten Medium und die Gewinnung des Proteins aus der Kultur umfaßt.

FIG. 1

# FIG. 2(I)

TGTTAACACCCTGGGTCAAAAATTGATATTTAGTAA

AATTACTTGCACTTTGTGCATTTTTTCATAAGATGAGTCATATGTTTTAAATTGTAGTAATGAAAAACAGTATTATATCATAATGAATTGGTACCTTAATAAAAGAGATGGAGGTAACTT

ATGGATAACAATCCGAACATCAATGAATGCATTCCTTATAATTGTTTAAGTAACCCTCAAGTAGAAGTATTAGGTGGAGAAAGAATAGAAACTGGTTACACCCCAATCGATATTTCCTTG
MetAspAsnAsnProAsnIleAsnGluCysIleProTyrAsnCysLeuSerAsnProGlnValGluValLeuGlyGlyGluArgIleGluThrGlyTyrThrProIleAspIleSerLeu

TGGCTAACGGCAATTTCTTTTGAGTGAATTTGTTCCCGGTGCTGGATTTGTGTTAGGACTAGTTGATATAATATGGGGAATTTTTGGTCCCTCTCAATGGGACGCATTTCTTGTACAAATT
SerLeuThrGlnPheLeuLeuSerGluPheValProGlyAlaGlyPheValLeuGlyLeuValAspIleIleTrpGlyIlePheGlyProSerGlnTrpAspAlaPheLeuValGlnIle

GAACAGTTAATTAACCAAAGAATAGAAGAATTCGCTAGGAACCAAGCCATTTCTAGATTAGAAGGACTAAGCAATCTTTATCAAATTTACGCAGAATCTTTTAGAGAGTGGGAAGCAGAT
GluGlnLeuIleAsnGlnArgIleGluGluPheAlaArgAsnGlnAlaIleSerArgLeuGluGlyLeuSerAsnLeuTyrGlnIleTyrAlaGluSerPheArgGluTrpGluAlaAsp

CCTACTAATCCAGCATTAAGAGAAGAGATGCGTATTCAATTCAATGACATGAACAGTGCCCTTACAACCGCTATTCCTCTTTTTGCAGTTCAAAATTATCAAGTTCCTCTTTTATCAGTA
ProThrAsnProAlaLeuArgGluGluMetArgIleGlnPheAsnAspMetAsnSerAlaLeuThrThrAlaIleProLeuPheAlaValGlnAsnTyrGlnValProLeuLeuSerVal

TATGTTCAAGCTGCAAATTTACATTTATCAGTTTTGACAGATGTTTCAGTGTTTGGACAAAGGTGGGGATTTGATGCCGCGACTATCAATAGTCGTTATAATGATTTAACTAGGCTTATT
TyrValGlnAlaAlaAsnLeuHisLeuSerValLeuArgAspValSerValPheGlyGlnArgTrpGlyPheAspAlaAlaThrIleAsnSerArgTyrAsnAspLeuThrArgLeuIle

GGCAACTATACAGATCATGCTGTACGCTGGTACAATACGGGATTAGAGCCGTGTATGGGGACCGGATTCTAGAGATTGGATAAGATATAATCAATTTAGAAGAGAATTAACACTAACTGTA
GlyAsnTyrThrAspHisAlaValArgTrpTyrAsnThrGlyLeuGluArgValTrpGlyProAspSerArgAspTrpIleArgTyrAsnGlnPheArgArgGluLeuThrLeuThrVal

TTAGATATCGTTTCTCTATTTCCGAACTATGATAGTAGAACGTATCCAATTCGAACAGTTTCCCAATTAACAAGAGAAATTTATACAAACCCACTATTAGAAAATTTTGATGGTAGTTTT
LeuAspIleValSerLeuPheProAsnTyrAspSerArgThrTyrProIleArgThrValSerGlnLeuThrArgGluIleTyrThrAsnProValLeuGluAsnPheAspGlySerPhe

CGTGCTCTGGCTCAGGGCATAGAAGGAAGTATTAGGAGTCCACATTTGATGGATATACTTAACAGTATAACCATCTATACGGATGCTCATAGAGGAGAATATTATTGGTCAGGGCATCAA
ArgAlaLeuAlaGlnGlyIleGluGlySerIleArgSerProHisLeuMetAspIleLeuAsnSerIleThrIleTyrThrAspAlaHisArgGlyGluTyrTyrTrpSerGlyHisGln

ATAATGGCTTCTCCTGTAGGGTTTTCGGGGCCAGAATTCACTTTTTCCGGTATATGGAACTATGGGAAATGCAGCTCCACAACAACGTATTGTTGCTCAACTAGGTCAGGGCGTGTATAGA
IleMetAlaSerProValGlyPheSerGlyProGluPheThrPheProLeuTyrGlyThrMetGlyAsnAlaAlaProGlnGlnArgIleValAlaGlnLeuGlyGlnGlyValTyrArg

ACATTATCGTCCACTTTATATAGAAGACCTTTTTAATATAGGGATAAATAATCAACAACTATCTGTTCTTCACGGGACAGAATTTGCTTATGGAACCTCCTCAAATTTGCCATCCGCTGTA
ThrLeuSerSerThrLeuTyrArgArgProPheAsnIleGlyIleAsnAsnGlnGlnLeuSerValLeuAspGlyThrGluPheAlaTyrGlyThrSerSerAsnLeuProSerAlaVal

TACAGAAAAAGCGGAACGGTAGATTCGCTGGATGAAATACCCGCCACAGAATAACAACGTGCCACCTAGGCAAGGATTTAGTCATCGATTAAGCCATGTTTCAATGTTTCGTTCAGGCTTT
TyrArgLysSerGlyThrValAspSerLeuAspGluIleProProGlnAsnAsnAsnValProProArgGlnGlyPheSerHisArgLeuSerHisValSerMetPheArgSerGlyPhe

# FIG. 2(2)

EP 0 224 331 B1

```
AGTAATAGTAGTGTAAGTATAATAAGAGTTCCTATGTTCTCTTGGATACATCGTAGTGTTGAATTTAATAATATAATTCCTTCATCACAAATTACACAAATACCTTTAAAAAAATTTAAT
SerAsnSerSerValSerIleIleArgAlaProMetPheSerTrpIleHisArgSerAlaGluPheAsnAsnIleIleProSerSerGlnIleThrGlnIleProLeuThrLysSerThr

AATCTTGGCTCTGGAACTTCTGTCGTTAAAGGACCAGGATTTACAGGAGGAGATATTCTTCGAAGAACTTCACCTGGCCAGATTTCAACCTTAAGAGTAAATATTACTGCACCATTATCA
AsnLeuGlySerGlyThrSerValValLysGlyProGlyPheThrGlyGlyAspIleLeuArgArgThrSerProGlyGlnIleSerThrLeuArgValAsnIleThrAlaProLeuSer

CAAAGATATCGGGTAAGAATTCGCTACGCTTCTACCACAAATTTACAATTCCATACATCAATTGACGGAAGACCTATTAATCAGGGGAATTTTTCAGCAACTATGAGTAGTCGGAGTAAT
GluArgTyrArgValArgIleArgTyrAlaSerThrThrAsnLeuGlnPheHisThrSerIleAspGlyArgProIleAsnGlnGlyAsnPheSerAlaThrMetSerSerArgSerAsn

TTACAGTCCGGAAGCTTTAGGACTGTAGGTTTTACTACTCCGTTTAACTTTTCAAATGGATCAAGTGTATTTACGTTAAGTGCTCATGTCTTCAATTCAGGCAATGAAGTTTATATAGAT
LeuGlnSerGlySerPheArgThrValGlyPheThrThrProPheAsnPheSerAsnGlySerSerValPheThrLeuSerAlaHisValPheAsnSerGlyAsnGluValTyrIleAsp

CGAATTGAATTTGTTCCGGCAGAAGTAACCTTTGAGGCAGAATATGATTTAGAAAGAGCACAAAAGGCGGTGAATGAGCTGTTTACTTCTTCCAATCAAATCGGGTTAAAAACAGATGTG
ArgIleGluPheValProAlaGluValThrPheGluAlaGluTyrAspLeuGluArgAlaGlnLysAlaValAsnGluLeuPheThrSerSerAsnGlnIleGlyLeuLysThrAspVal

ACGGATTATCATATTGATCAAGTATCCAATTTAGTTGAGTGTTTATCTGATGAATTTTGTCTGGATGAAAAAAAAGAATTGTCCGAGAAAGTCAAACATGCGAAGCGACTTAGTGATGAG
ThrAspTyrHisIleAspGlnValSerAsnLeuValGluCysLeuSerAspGluPheCysLeuAspGluLysLysGluLeuSerGluLysValLysHisAlaLysArgLeuSerAspGlu

CGGAATTTACTTCAAGATCCAAACTTTAGAGGGATCAATAGACAACTAGACCGTGGCTGGAGAGGAAGTACGGATATTACCATCCAAGGAGGCGATGACGTATTCAAAGAGAATTACGTT
ArgAsnLeuLeuGlnAspProAsnPheArgGlyIleAsnArgGlnLeuAspArgGlyTrpArgGlySerThrAspIleThrIleGlnGlyGlyAspAspValPheLysGluAsnTyrVal

ACGCTATTGGGTACCTTTGATGAGTGCTATCCAACGTATTTATATCAAAAAATAGATGAGTCGAAATTAAAAGCCTATACCCCGTTACCAATTAAGAGGGTATATCGAAGATAGTCAAGAC
ThrLeuLeuGlyThrPheAspGluCysTyrProThrTyrLeuTyrGlnLysIleAspGluSerLysLeuLysAlaTyrThrArgTyrGlnLeuArgGlyTyrIleGluAspSerGlnAsp

TTAGAAATCTATTTAATTCGCTACAATGCCAAACACGAAACAGTAAATGTGCCAGGTACGGGGTTCCTTATGGCCGCTTTCAGCCCCAAGTCCAATCGGAAAATGTGCCCATCATTCCCAT
LeuGluIleTyrLeuIleArgTyrAsnAlaLysHisGluThrValAsnValProGlyThrGlySerLeuTrpProLeuSerAlaProSerProIleGlyLysCysAlaHisHisSerHis

CATTTCTCCTTGGACATTGATGTTGGATGTACAGACTTAAATGAGGACTTAGGTGTATGGGTGATATTCAAGATTAAGACGCAAGATGGCCATGCAAGACTAGGAAATCTAGAATTTCTC
HisPheSerLeuAspIleAspValGlyCysThrAspLeuAsnGluAspLeuGlyValTrpValIlePheLysIleLysThrGlnAspGlyHisAlaArgLeuGlyAsnLeuGlnPheLeu

GAAGAGAAACCATTAGTAGGAGAAGCACTAGCTCGTGTGAAAAGAGCCGAGAAAAAATGGAGAGACAAACGTGAAAAATTGGAATGGGAAACAATATTGTTTATAAAGAGGAAAAGAA
GluGluLysProLeuValGlyGluAlaLeuAlaArgValLysArgAlaGluLysLysTrpArgAspLysArgGlnLysLeuGluTrpGluThrAsnIleValTyrLysGluAlaLysGlu

TCTGTAGATGCTTTATTTGTAAACTCTCAATATGATAGATTACAAGCGGATACCAACATCGCCCATGATTCATGCGGCAGATAAACGCGTTCATAGCATTCGAGAAGCTTATCTGCCAGAG
SerValAspAlaLeuPheValAsnSerGlnTyrAspArgLeuGlnAlaAspThrAsnIleAlaHisIleHisAlaAlaAspLysArgValHisSerIleArgGluAlaTyrLeuProGlu
```

EP 0 224 331 B1

```
      2770      2780      2790      2800      2810      2820      2830      2840      2850      2860      2870      2880
CTGTCTGTGATTCCGGGTGTCAATGCGGCTATTTTTGAAGAATTAGAAGGCCGTATTTTCACTGCATTCTCCCTATATGATGCGGAGAAATGTCACTAAAAATGGTGATTTTAATAATGGC
LeuSerValIleProGlyValAsnAlaAlaIlePheGluGluLeuGluGlyArgIlePheThrAlaPheSerLeuTyrAspAlaArgAsnValIleLysAsnGlyAspPheAsnAsnGly

      2890      2900      2910      2920      2930      2940      2950      2960      2970      2980      2990      3000
TTATCCTGCTGGAACGTGAAAGGGCATGTAGATGTAGAAGAACAAAACAACCAACGTTCGGTCCTTGTTGTTCCGGAATGGGAAGCAGAAGTGTCACAAGAAGTTCGTGTCTGTCCGGGT
LeuSerCysTrpAsnValLysGlyHisValAspValGluGluGlnAsnAsnGlnArgSerValLeuValValProGluTrpGluAlaGluValSerGlnGluValArgValCysProGly

      3010      3020      3030      3040      3050      3060      3070      3080      3090      3100      3110      3120
CGTGGCTATATCCTTCGTGTCACAGCCGTACAAGGAGGGATATGGAGAAGGTTGCCTAACCATTCATGAGATCGAGAACAATACAGACGAACTGAAGTTTAGCAACTTTGTAGAAGAGGAA
ArgGlyTyrIleLeuArgValThrAlaTyrLysGluGlyTyrGlyGluGlyCysValThrIleHisGluIleGluAsnAsnThrAspGluLeuLysPheSerAsnPheValGluGluGlu

      3130      3140      3150      3160      3170      3180      3190      3200      3210      3220      3230      3240
GTATATCCAAACAACACGGTAACGTGTAATGATTATACTGCGACTCAAGAACAATATGCGGGGTACCTACACTTCTCGTAATCGAGGATATGACGGAGCCTATGAAAGCAATTCTTCTGTA
ValTyrProAsnAsnThrValThrCysAsnAspTyrThrAlaThrGlnGluGlnTyrGluGlyThrTyrThrSerArgAsnArgGlyTyrAspGlyAlaTyrGluSerAsnSerSerVal

      3250      3260      3270      3280      3290      3300      3310      3320      3330      3340      3350      3360
CCAGCTGATTATGCATCAGCCTATGAAGAAAAAGCATATACAGATGGACGAAGAGACAATCCTTGTGAATCTAACAGAGGATATGGGGATTACACACCACTACCAGCTGGCTATGTGACA
ProAlaAspTyrAlaSerAlaTyrGluGluLysAlaTyrThrAspGlyArgArgAspAsnProCysGluSerAsnArgGlyTyrGlyAspTyrThrProLeuProAlaGlyTyrValThr

      3370      3380      3390      3400      3410      3420      3430      3440      3450      3460      3470      3480
AAAGAATTAGAGTACTTCCCAGAAACCGATAAGGTATGGATTGAGATCGGAGAAACGGAAGGAACATTCATCGTGGACAGCGTGGAATTACTTCTTATGGAGGAATAATATATGCTTTAA
LysGluLeuGluTyrPheProGluThrAspLysValTrpIleGluIleGlyGluThrGluGlyThrPheIleValAspSerValGluLeuLeuLeuMetGluGlu***
```

14

# FIG. 3

├──┤ 1kb

Bm       Hd   Hd Pv       Ps    Bg    Ps Pv   Hd     Bg    Bm

├─┤ 100 bp

Ah Cl Ec      Ec   Cl   Ec Hd     Kp       Hd      Pv Ah

Ps: PstI   Ec: EcoRI   Bm: BamHI

Pv: PvuII   Bg: BglII   Hd: HindIII

Cl: ClaI   Kp: KpnI   Ah: AhaIII

EP 0 224 331 B1

FIG. 4

(1)

(2)

(3)